# EUROPEAN PATENT SPECIFICATION

(11) **EP 2 758 035 B1**
(45) Date of publication and mention of the grant of the patent: **12.01.2022**
(21) Application number: 12772735.2
(22) Date of filing: 19.09.2012
(51) Int. Cl.: A61K 9/127, A61K 47/36

(54) **LIPOSOME PRODUCTION USING ISOPROPANOL**
LIPOSOMHERSTELLUNG MIT ISOPROPANOL
PRODUCTION DE LIPOSOME À L'AIDE D'ISOPROPANOL

(30) Priority: 20.09.2011 GB 201116248
(43) Date of publication of application: 30.07.2014
(73) Proprietor: GlaxoSmithKline Biologicals S.A., 1330 Rixensart (BE)
(72) Inventor: DE KESEL, Carine, B-1330 Rixensart (BE); MANCUSO, Vincent, B-1330 Rixensart (BE)
(74) Representative: Thornley, Rachel Mary
(86) International application number: PCT/EP2012/068448
(87) International publication number: WO 2013/041572

(56) References cited:
- WO-A1-94/03157
- WO-A1-03/043729
- WO-A2-02/066490
- WO-A2-2008/009650
- US-A1- 2010 226 973

## Description

### Summary of invention

The present invention relates to methods for the production of liposomes. In particular, the invention relates to methods for the production of cholesterol containing liposomes using isopropanol.

### Background to the invention

Immunologically active saponin fractions having adjuvant activity derived from the bark of the South American tree Quillaja Saponaria Molina are known in the art. For example QS21, also known as QA21, an Hplc purified fraction from the Quillaja Saponaria Molina tree and it's method of its production is disclosed (as QA21) in US patent No. 5,057,540. Quillaia saponin has also been disclosed as an adjuvant by Scott et al, Int. Archs. Allergy Appl. Immun., 1985, 77, 409. However, the use of QS21 as an adjuvant is associated with certain disadvantages. For example when QS21 is injected into a mammal as a free molecule it has been observed that necrosis, that is to say, localised tissue death, occurs at the injection site.

It has now surprisingly been found that necrosis at the injection site can be avoided by use of formulations containing a combination of QS21 and a sterol. Preferred sterols include -sitosterol, stigmasterol, ergosterol, ergocalciferol and cholesterol. These sterols are well known in the art, for example cholesterol is disclosed in the Merck Index, 11th Edn., page 341, as a naturally occuring sterol found in animal fat.

During the last few decades, liposomes (also known as bilayer lipid vesicles) are increasingly being used to encapsulate and deliver pharmaceutical compounds. Liposomes consist of one or more lipid and/or phospholipid bilayers and can contain other molecules, such as proteins or carbohydrates, in their structure. The lipidic layer on the liposome confines and protects the enclosed pharmaceutical compound until the liposome reaches its destination and adheres to the outer membrane of target cancer cells. By this process, drug toxicity to healthy cells is minimized and therapeutic efficacy can be increased. Due to the presence of both lipid and aqueous phases in their structure, liposomes can be utilized in the encapsulation or entrapment of water- and lipid-soluble material in addition to the amphiphilic compounds.

The manufacture of liposomes requires input of energy to a dispersion of lipid/phospholipid molecules in an aqueous medium. The underlying mechanism for the formation of liposomes and is the hydrophilic-hydrophobic interaction between phospholipids and water molecules. Most methods of liposome preparation, involve solubilisation of the ingredients in organic solvents, such as chloroform and methanol.

The inventors found a number of problems associated with the large scale production of liposomes comprising cholesterol. The present invention addresses these problems.

### Summary of the invention

The initial step in the production of a liposome is the preparation of a lipidic film. This process involves solubilising a lipid mix in an organic solvent to form a homogeneous mix and subsequently removing the solvent from the mixture so that a lipidic film is formed on the surface of the reaction container in which the mix is contained.

The inventors found that if a lipid mix comprising cholesterol was dissolved in ethanol, precipitation of the homogeneous mix occurred before all of the ethanol could be removed. Once precipitation has occurred it is very difficult to fully dry the resulting mixture because the components form a "sludge" from which solvent removal becomes almost impossible. The resulting precipitate mixture has to be discarded causing a commercial loss.

The inventors have found that by replacing ethanol with isopropanol the precipitation can be avoided. The invention therefore provides a method for producing a lipidic film comprising the steps of: (i) dissolving a lipid mix in isopropanol to form a homogeneous mix; and (ii) removing the solvent from the homogeneous mix to form a lipidic film, wherein the lipid mix comprises a lipid and cholesterol.

The method allows for the fast, efficient production of cholesterol comprising liposomes. In addition, the method has a number of further advantages

Firstly, as described above, the method avoids precipitation of cholesterol during formation of the lipidic film. Secondly, the total cycle time for lipidic film formation is reduced. Therefore degradation of the components of the lipid mix is reduced when drying the lipid mix. Finally, it is essential to work with an organic solvent with low toxicity as the residual solvent present in the lipidic film is essential to allow the hydration of the lipidic film during the industrial manufacturing scale.

Although not wishing to be bound by this theory, the inventors believe that the reduced temperature of the homogenous reaction mixture and reduced solvent removal time are caused by the increased volatility of isopropanol when compared to other organic solvents. Because of the reduced temperature and solvent removal time, any additional components of the lipid mix which may be unstable at higher temperatures, for example monophosphoryl lipid A (MPL), are exposed to a lower temperature and are exposed to heating for less time, which is consequently beneficial for their stability.

Secondly, when producing liposomes, it is normal for there to be a residual amount of solvent, used to dissolve the lipid mix, left in the final liposome formulation. The residual amount of solvent is crucial for the rehydration of the lipidic film and prevents the liposomes sticking to the sides of the reaction vessel upon rehydration of the lipidic film. However, these residual solvents, known as organic volatile impurities (OVIs), have no therapeutic benefits but can be hazardous to human health and the environment (Dwivedi, 2002). It is therefore desirable for the organic solvent to have no or at least a low toxicity. The isopropanol used in the methods of the present invention is a class 3 solvent which has a low toxicity and is therefore considered a low risk to human health when associated with the liposomes produced using the methods of the invention.

In a first aspect of the present invention, there is provided a method for producing a lipidic film comprising the steps of:
(i) dissolving a lipid mix in isopropanol to form a homogenous mix, and
(ii) removing the isopropanol from the homogenous mix to form a lipidic film,
wherein the lipid mix comprises a lipid, cholesterol and a lipopolysaccharide, wherein the total amount of lipid mix dissolved in isopropanol is 200g or more.

In one embodiment the lipopolysaccharide is MPL.

The isopropanol may be removed from the homogeneous mix by vapour distillation. Alternatively, the isopropanol may be removed from the homogeneous mix by evaporation vacuum distillation, or by spray drying the homogenous mix

The invention also provides a method for producing liposomes comprising: a) producing a lipidic film according to the methods of the invention; b) hydrating the lipidic film with a hydrating solution to form a coarse liposome suspension; c) reducing size of the coarse liposome suspension produced in step (b) with high shear and high pressure homogenizer to form liposomes.

In one embodiment, the solution used to hydrate the lipidic film comprises a buffer. In another embodiment the buffer is a phosphate buffer.

The present disclosure further describes a method, wherein step c) comprises steps: c') pre-homogenising the coarse liposome suspension solution with a high shear mixer; and c") homogenising the solution produced in step c') with a high pressure homogeniser.

In one embodiment, the method comprises an additional step d) sterilising the liposomes.

The present disclosure further describes a lipidic film or a liposome produced by the methods of the invention.

In a further aspect, the present disclosure describes a liposome comprising cholesterol and isopropanol, wherein the isopropanol will be present as a residual amount.

### Detailed description of the invention

### Step (i)

Step (i) of the method of the invention involves dissolving a lipid mix in isopropanol to form a homogeneous mix. This step may take place in a first reaction container. Suitable reaction containers for use in the invention can hold a volume of liquid and include, but are not limited to, reactor vessels, evaporator flasks and test tubes.

### Lipid Mix

The lipid mix used in step (i) comprises at least one lipid, but may comprise 2, 3, 4, 5, or more different lipids. In one embodiment, the lipids are phospholipids. In another embodiment the lipid is dioleoylphosphatidylcholine (DOPC).

The amount of lipid present in the lipid mix will usually be in the range of about 40 to 90% w/w, e.g. about 40, 41, 42, 43, 44, 45, 46, 47, 48, 49, 50, 51, 52, 53, 54, 55, 56, 57, 58, 59, 60, 61, 62, 63, 64, 65, 67, 68, 69, 70, 71, 72, 73, 74, 75, 76, 77, 78, 79, 80, 81, 82, 83 84, 85 86, 87, 88, 89 or 90. For example, the amount of lipid present in the lipid mix may be 77%.

### Cholesterol

The lipid mix also comprises cholesterol. Cholesterol is a waxy steroid metabolite found in the cell membranes and transported in the blood plasma of all animals. It is an essential structural component of mammalian cell membranes, where it is required to establish proper membrane permeability and fluidity. Cholesterol has the formula (C₂₇H₄₆O) and is also known as (3β)-cholest-5-en-3-ol. Cholesterol is white crystalline powder with a molar mass of 386.65 g/mol and a solubility in water of 0.095 mg/L (30 °C).

The amount of cholesterol present in the lipid mix will usually be in the range of about 5 to 40% w/w, e.g. about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40. For example, the amount of cholesterol present in the lipid mix may be 19.2%.

### Lipopolysaccharide

The lipid mix further comprises a lipopolysaccharide, an immunostimulant which is a TLR-4 agonist. The lipopolysaccharide is intended to function as an immunostimulant in liposomes generated using the methods of the inventions.

In one embodiment the lipopolysaccharide is a non-toxic derivative of lipid A, such as monophosphoryl lipid A or more particularly 3-Deacylated monophoshoryl lipid A (3D-MPL). 3D-MPL is sold under the name MPL by GlaxoSmithKline Biologicals S.A., and is referred throughout the document as MPL or 3D-MPL. See, for example, US Patent Nos. 4,436,727; 4,877,611; 4,866,034 and 4,912,094. 3D-MPL primarily promotes CD4+ T cell responses with an IFN-y (Thl) phenotype. 3D-MPL can be produced according to the methods disclosed in GB2220211 A. Chemically it is a mixture of 3-deacylated monophosphoryl lipid A with 3, 4, 5 or 6 acylated chains. Small particle 3D-MPL can be used in the methods of the invention. Small particle 3D-MPL has a particle size such that it can be sterile-filtered through a 0.22µm filter. Such preparations are described in WO94/21292.

Other TLR-4 ligands which can be used are alkyl Glucosaminide phosphates (AGPs) such as those disclosed in WO 98/50399 or US Patent No. 6,303,347 (processes for preparation of AGPs are also disclosed), suitably RC527 or RC529 or pharmaceutically acceptable salts of AGPs as disclosed in US Patent No. 6,764,840. Some AGPs are TLR-4 agonists, and some are TLR-4 antagonists. Both are thought to be useful as immunostimulants.

The amount of lipopolysaccharide present in the lipid mix if included will usually be in the range of about 0.5 to 10% w/w, e.g. about 0.5, 0.6, 0.7, 0.8, 0.9, 1.0, 1.25, 1.5, 1.75, 2, 2.2.5, 2.5, 2.75, 3.0, 3.5, 4.0, 4.5, 5.0, 6, 7, 8, 9 or 10%. For example, the amount of lipopolysaccharide present in the lipid mix may be 3.8%.

The various components of the lipid mix, as described above, will usually be in a dry powdered form. However, the methods of the invention are also intended to include components which are in liquid form, e.g. oils or solutions.

### Scale

Although the inventors found that precipitation of a lipid mix comprising cholesterol, dissolved in ethanol, occurred at both small and large scale, they found that it was particularly a problem when producing lipidic films on a large scale. Therefore, in one embodiment, the total amount of lipid mix dissolved in isopropanol is 200g or more, e.g. 200, 250, 300, 350, 400, 500, 600, 700, 800, 900, 1000, 2000, 3000, 4000, 5000, 7500, 10000g or more.

### Isopropanol

The methods of the invention require that the lipid mix is dissolved in isopropanol (CAS 67-63-0). Isopropanol (C₃H₈0), also known as isopropyl alcohol, propan-2-ol, or 2-propanol, is a colourless, flammable chemical compound with a strong odour. It is the simplest example of a secondary alcohol, where the alcohol carbon is attached to two other carbons sometimes shown as (CH₃)₂CHOH. It is a structural isomer of propanol. Isopropanol has a molar mass of 60.1 g mol⁻¹, a density of 0.786 g/cm³ (20 °C) and a boiling point of 82.5°C (356 K).

Described herein are methods wherein each kilogram of lipid mix will be dissolved in a volume of isopropanol in the range of about 0.5 to 5 litres, e.g. about 0.5, 0.75, 1.0, 1.25, 1.5, 1.75, 2.0, 2.5, 3.0, 3.5, 4.0, or 5.0 litres.

### Homogeneous mix

The dissolving required in step (i) is intended to ensure a uniform composition throughout the solution. This may be achieved by any suitable means including, but not limited to, agitation,
mechanical or manual stirring or repeat pipetting.

### Step (ii)

Step (i) of the method of the invention involves removing the isopropanol from the homogenous mix to form a lipidic film.

The isopropanol may be removed by vapour distillation. Vapour distillation is well known in the art and involves heating the first reaction container to a pre-determined temperature causing the isopropanol to evaporate from the homogeneous mix. The solution may be under a vacuum in order to facilitate evaporation. In one embodiment, the isopropanol is removed using a rotary evaporator, for example the Rotavapor as supplied by Buchi in combination with an evaporating flask. As the name suggests, a rotary evaporator turns the first reaction container during the removal/heating process allowing for more efficient removal of the isopropanol. The evaporating flask used in the methods of the invention will usually be in the range of about 0.2 to 50 litres in volume, e.g. about 0.2, 0.5, 1.0, 2, 3, 4, 5, 7, 10, 15, 20, 25, 30, 40 or 50 litres.

During the removal of the isopropanol, the first reaction container will usually be heated to a temperature in the range of about 20°- 100°C, e.g. about 20, 22, 25, 30, 35, 40, 45, 50, 55, 60, 65, 70, 75, 80, 85, 90, 95 or 100°C. For example, the first reaction container may be heated to 55°C. The first reaction container may be heated in a water bath.

### Lipidic film

As the isopropanol evaporates from the first reaction container the lipids begin to form a lipidic film on the surface of the reaction vessel. This phase is also known as the homogeneous oil phase.

The lipid mix used in the methods of the invention also comprises cholesterol. Therefore, the lipidic film will also include cholesterol. In addition, the lipidic film may also include any additional components.

As described above, it is advantageous for a residual amount of the isopropanol to remain in the lipidic film and thus the first reaction container. This residual amount plays an important role in the hydration of the lipidic film. The methods of the invention therefore may include the total removal of the isopropanol. However, the methods of the invention are intended to include the retention of a proportion of the original isopropanol in the first reaction container after step (ii). The amount of isopropanol retained in the first reaction container will usually be in the range of about 0.01 % and 20% of the initial isopropanol used, e.g. about 0.01, 0.02, 0.03, 0.05, 0.1, 0.2, 0.3, 0.5, 1.0, 2.0, 3.0, 4.0, 5.0, 7.5, 10, 12.5, 15, 17.5 or 20%.

### Liposomes

The lipidic films produced by the methods of the invention are primarily intended to be used for the production of liposomes. The lipidic film is hydrated with a hydrating solution and the resulting mixture is then homogenised to form liposomes.

The term "liposomes" is well known in the art and defines a general category of vesicles which comprise one or more lipid bilayers surrounding an aqueous space. Liposomes produced by the methods of the invention can have a diameter between about 10 and 300nm, e.g. about 10, 20, 30, 40, 50, 75, 100, 125, 150, 175, 200, 225, 250, 275 and 300nm. Liposomes produced by the methods of the invention may have a diameter between 50 to 150nm. The diameter of the liposomes can be affected, for example, by extrusion of the liposomal composition through sieves or meshes with a known pore size. This and further methods of controlling the size of liposomes are well known in the art and are described, for example, in Mayhew et al (1984) Biochim. Biophys. Acta 775:169-174 or Olson et al. (1979) Biochim. Biophys. Acta 557:9-23.

### Hydrating solution

The hydrating solution used to hydrate the lipidic film will usually comprise a buffer. In one embodiment the buffer is a phosphate based buffer. The hydrating solution will usually have a pH of about 5 to 9, e.g. about 5, 5.25, 5.5, 5.75, 6, 6.25, 6.5, 6.75, 7, 7.25, 7.5, 7.75, 8, 8.25, 8.5, 8.75 or 9.

The hydrating solution is added to the first reaction container comprising the lipidic film. The addition of the hydrating solution is intended to ensure suspension of the lipidic film and its removal from the walls of the first reaction container. This may be achieved by any means including, but not limited to, agitation, mechanical or manual stirring. The resultant solution is termed the coarse liposome suspension.

The coarse liposome suspension may be pre-homogenised with a high shear mixer. This step is intended to reduce the size of the coarse liposomes. A rotor or impellor, together with a stationary component known as a stator, or an array of rotors and stators, is used either in a tank containing the solution to be mixed, or in a pipe through which the solution passes, to create shear. A high shear mixer can be used to create emulsions, suspensions, lyosols (gas dispersed in liquid) and granular products.

The pre-homogenised coarse liposome suspension may be further homogenized with a high pressure homogenizer. This process is well known in the art and usually involves a standard homogeniser. The solution may be homogenised in the first reaction container or the solution may be transferred to a second reaction container before homogenising. Suitable reaction containers for use as the second reaction container can hold a volume of liquid and include, but are not limited to tanks, such as stainless steel tanks, flasks or beakers. The coarse liposome suspension may be pressurised during homogenisation.

For example, the coarse liposome suspension may be homogenised using a high shear homogenizer in-line with a high pressure homogeniser. Such technology is well known in the art and homogenisation usually occurs in a range of pressure between 5000 and 30000psi, i.e. 5000, 6000, 7000, 8000, 9000, 10000, 11000, 12000, 13000, 14000, 15000, 16000, 17000, 18000, 19000, 20000, 21000, 22000, 23000, 24000, 25000, 26000, 27000, 28000, 29000 or 30000 psi.

### Sterilisation

In one embodiment the liposomes produced by the methods of the invention are sterilised. In one embodiment, the liposomes are sterilised by filtration. Alternatively, the liposomes may be sterilised by irradiation, in particular gamma irradiation.

### Lipidic film/Liposome

The present disclosure also describes a lipidic film, or liposome produced by the methods of the invention. These lipidic films and liposomes are distinguishable from those known in the art as they comprise cholesterol and residual isopropanol. Accordingly the present disclosure also describes a liposome comprising cholesterol and isopropanol.

The residual isopropanol may be present at 20-2000µ9 IPA/ml of liposomes, e.g. 50-1900 µg IPA/ml of liposomes, 100-1800 µg IPA/ml of liposomes, 150-1500 µg IPA/ml of liposomes, 200-1300 µg IPA/ml of liposomes, 250-1200 µg IPA/ml of liposomes, 300-1100 µg IPA/ml of liposomes, 350-1000 µg IPA/ml of liposomes, or 400-900 µg IPA/ml of liposomes.

The liposomes described herein may further comprise additional immunostimulants or an antigen or antigenic preparation.

For example, said one or more immunostimulants may be a saponin.

### Immunogenic composition

The present disclosure also describes an immunogenic composition comprising a liposome produced by the methods of the invention. For example, the immunogenic composition may consist essentially of either a single liposomal population or a mixture of liposomal populations, and an antigen or antigenic preparation.

The antigen may be a bacterial, viral or cancer antigen.. The antigen may be a recombinant protein. The recombinant protein may be a prokaryotic protein.

The antigen may comprise or consist of WT-1 expressed by the Wilm's tumor gene, or its N-terminal fragment WT-IF comprising about or approximately amino acids 1-249. WTI is a protein originally found to be overexpressed in paediatric kidney cancer, Wilm's Tumor. An antigen that may be used comprises nearly the full length protein as antigen. For example, the antigen may comprise or consist of the WTI-AIO protein, which is a 292 AA recombinant fusion protein consisting of a 12mer truncated tat sequence and amino acids number 2 - 281 of the WTI sequence.

PRAME (also known as DAGE) is another antigen that may be used as the tumour associated antigen of the present invention.

The antigen and its preparation are described in US patent No. 5, 830, 753. PRAME is found in the Annotated Human Gene Database H-Inv DB under the accession numbers: U65011.1, BC022008.I, AK129783.I, BC014974.2, CR608334.I, AF025440.I, CR591755.I, BC039731.1, CR623010.I, CR611321.1, CR618501.1, CR604772.I, CR456549.I, and CR620272.I.

Fusion proteins that comprise the PRAME antigen may also be used. PRAME or a fragment or derivative thereof may be employed, optionally in the form of a fusion protein with a heterologous fusion partner. In particular, PRAME antigen may suitably be employed in the form of a fusion protein with *Haemophilus influenzae B* protein D or a portion thereof or derivative thereof. The portion of protein D that may be employed suitably does not include the secretion sequence or signal sequence. Suitably the fusion partner protein comprises amino acids Met-Asp-Pro at or within the N-terminus of the fusion protein sequence and in which the fusion partner protein does not include the secretion sequence or the signal sequence of protein D. For example the fusion partner protein may comprise or consist of approximately or exactly amino acids 17 to 127, 18 to 127, 19 to 127 or 20 to 127 of protein D. Suitable PRAME antigens based on fusions proteins with protein Dare are described in WO2008/087102.

NY-ESO-1 is another antigen that may be used as the tumour associated antigen of the present invention. NY-ESO-1 or a fragment or derivative thereof may be employed, optionally in the form of a fusion protein with a heterologous fusion partner. NY-ESO-1 is described in US5804381. The protein NY-ESO-1 is approximately 180 amino acids in length and can be described as being composed of three regions: (a) an N- terminal region being about amino acids 1-70 (b) a central region being about amino acids 71-134 and a C terminal region being about amino acids 71-180. NY-ESO-1 may be employed as a fusion protein for example as a fusion with LAGE-1 which is a further CT antigen, or a fragment thereof. Where fragments of NY-ESO-1 are employed these suitably include one or more MHC Class 1 or Class 2 epitopes e.g. those known as A31, DRI, DR2, DR4, DR7, DP4, B35, B51, Cw3, Cw6 and A2 (see WO2008/089074).

A further antigen that may be employed in accordance with the present invention is a MAGE antigen, e.g. ofthe MAGE-3 family such as MAGE-A3. MAGE-3 antigens have, for example, been described as suitable to be formulated in combination with NY-ESO-1 - see WO2005/105139.

MAGE antigens such as MAGE-A3 may be used as such or in the form of a derivative e.g. a chemically modified derivative and/or in the form of a fusion protein with a heterologous fusion partner. For example the MAGE antigen may contain reduced disulphide bridges to form free thiols which have been derivatised eg with carboxamide or carboxymethyl groups, see WO99/40188. In particular, MAGE antigens may suitably be employed in the form of a fusion protein with *Haemophilus influenzae B* protein D or a portion thereof or derivative thereof. For example approximately the first third of protein D or the N-terminal 100 to 110 amino acids of protein D may be employed as the fusion partner, see WO99/40188.

The antigen may comprise or consist of preparations derived from parasites that cause Malaria such as Plasmodium falciparum or Plasmodium vivax.

Possible antigens derived from Plasmodium falciparum include circumsporozoite protein (CS protein), RTS, PfEMP-1, Pfs 16 antigen, MSP-1, MSP-3, LSA-1, LSA-3, AMA-I and TRAP. Other P. falciparum antigens include EBA, GLURP, RAPI, RAP2, Sequestrin, Pf332, STARP, SALSA, PfEXPI, Pfs25, Pfs28, PFS27/25, Pfs48/45, Pfs230 and their analogues in other Plasmodium spp. The antigen may be an entire protein or an immunogenic fragment thereof.

An antigen derived from Plasmodium falciparum CS protein may be in the form of a hybrid fusion protein. The fusion protein may contain protein derived from P. falciparum CS protein fused to another protein or fragment thereof. The fusion protein may contain an N-terminal or C-terminal fragment from the CS protein of P. falciparum. Alternatively, or in addition, the fusion protein may comprise one or more repeat units (e.g.I, 2, 3, 4, 5, 6, 7, 8, 9 or more repeat units) from the central region of P. falciparum CS protein. The fusion protein may also be a hybrid fusion protein comprising an antigen derived from CS protein together with a surface antigen from hepatitis B (HBsAg) or an immunogenic fragment thereof. Typically, the surface antigen from Hepatitis B comprises the major surface protein known as the S antigen, for example, S antigen derived from an adw serotype.

In particular, the fusion protein may comprise substantially all the C-terminal portion of the CS protein of P. falciparum, four or more tandem repeats of the CS protein immunodominant region, and the surface antigen from hepatitis B (HBsAg). In one aspect the fusion protein comprises a sequence which contains at least 160 amino acids which is substantially homologous to the C-terminal portion of the CS protein. In particular "substantially all" the C terminal portion of the CS protein includes the C terminus devoid of the hydrophobic anchor sequence. The CS protein may be devoid of the last 12 to 14 (such as 12) amino-acids from the C terminal.

For example, the fusion protein which may be used in the invention is a protein which comprises a portion of the CS protein of P. falciparum substantially as corresponding to amino acids 207 395 of P. falciparum 3D7 clone, derived from the strain NF54 (Caspers et al, supra) fused in frame via a linear linker to the N terminal of HBsAg. The linker may comprise part or all of the preS2 region from HBsAg.

A particular fusion protein for use in the invention is the fusion protein known as RTS, as described in WO 93/10152 and WO 98/05355. The RTS may be in the form of RTS,S mixed particles (wherein "S" represents an unfused monomer) or as RTS. The RTS,S particles comprise two polypeptides RTS and S that may be synthesized simultaneously and spontaneously form composite particulate structures (RTS,S) e.g. during purification. These particles may also be referred to a Virus Like Particles (VLP). Such particles can be prepared in a number of ways, for example by expressing the fusion protein in a suitable host such as yeast or bacteria.

It is believed that the presence of the surface antigen from Hepatitis B and the formation of the RTS,S particles boosts the immunogenicity of the CS protein portion of the hybrid protein, aids stability, and/or assists reproducible manufacturing of the protein.

The CS antigens may be used in conjunction with another antigen selected from any antigen which is expressed on the sporozoite or the pre-erythrocytic stage of the parasite life cycle such as the liver stage, for example liver stage antigen-1 (LSA-1), liver stage antigen-3 (LSA-3), thrombospondin related anonymous protein (TRAP), merozoite surface protein-1 (MSPI) the major merozoite surface protein, and apical merezoite antigen-1 (AMA-1). Other suitable antigens to use in conjunction with CS antigens include PfEMP-1, Pfs 16 antigen, MSP-3, LSA-3, AMA-I, TRAP, GLURP, RAPI, RAP2, Sequestrin, Pf332, STARP, SALSA, PfEXPI, Pfs25, Pfs28, PFS27/25, Pfs48/45, Pfs230.

Immunogenic fragments of any of the antigens as described herein will contain at least one epitope of the antigen and display malaria antigenicity and are capable of raising an immune response when presented in a suitable construct, such as for example when fused to other malaria antigens or other non-malaria antigens, or presented on a carrier, the immune response being directed against the native antigen. Typically the immunogenic fragments contain at least 20, or at least 50, or at least 100 contiguous amino acids from the malaria antigen.

Possible antigens from P. vivax include circumsporozoite protein (CS protein) based antigens and Duffy antigen binding protein and fragments thereof, such as PvRII (see eg WO02/12292).

Possible CS protein based antigens may include a fusion protein comprising sequences derived from a CS protein of P.vivax. For example, the fusion protein may be a hybrid fusion protein. The hybrid protein herein may contain protein derived from P. vivax type I and type II. In particular, the hybrid fusion protein may contain protein derived from P. vivax type I and type II fused to another protein or fragment thereof.

In one aspect of the disclosure, the hybrid fusion protein may comprise a hybrid protein derived from the CS proteins of P. vivax (CSV) and a surface antigen from Hepatitis B, generally the major surface protein known as the S antigen, such as the S antigen derived from an adwserotype.

Preferably, the fusion protein is an immunogenic hybrid fusion protein comprising: a. at least one repeat unit derived from the central repeat section of a type I circumsporozoite protein of P. vivax, b. at least one repeat unit derived from the central repeating section of a type II circumsporozoite protein of P. vivax, and c. surface antigen S derived from Hepatitis B virus.

The CSV derived antigen component of the invention is generally fused to the amino terminal end of the S protein. More specifically the C- terminus end of the CSV fragment is fused the N-terminus of said S antigen.

For example, a suitable fusion protein is CSV-S, as described in WO2008/009652.

In yeast cells once expressed, the hybrid fusion protein (comprising S antigen), is able to spontaneously assemble into a lipoprotein structure/particle composed of numerous monomers of said proteins (or VLPs). Such particles can be prepared by expressing the fusion protein in a suitable host such as yeast or bacteria.

When the chosen recipient yeast strain also carries in its genome one or more integrated copies of a hepatitis B S expression cassette, the resulting strain synthesizes hybrid protein as a fusion proteins, and also non- fused S antigen. These may spontaneously be assembled into lipoprotein particles comprising monomers of the hybrid fusion protein and monomers of the S antigen.

Also described, is a VLP comprising CSV-S and/or RTS units. The particle may consist essentially of CSV-S and RTS units. Alternatively, the particles produced may comprise or consist essentially of CSV-S, RTS and S units. Such mixed particles are described for exmaple in WO2008/009650.

Antigens of interest in the field of tuberculosis include Mtb72f and variants thereof, such as disclosed in WO2006117240. An antigen of particular interest is M72, the polypeptide sequence of which being provided in Seq ID No: 4 and a polynucleotide sequence encoding said polypeptide being provided in Seq ID No: 3 of WO2006117240.

A further antigen that may be employed in accordance with the present invention is the tuberculosis antigen Rv1753 and variants thereof, such as disclosed in WO2010010180, for example a Rv1753 sequence selected from Seq ID Nos: 1 and 2-7 of WO2010010180, in particular Seq ID No: 1. Another antigen of interest in the field of tuberculosis is Rv2386 and variants thereof, such as disclosed in WO2010010179, for example a Rv2386 sequence selected from Seq ID Nos: 1 and 2-7 of WO2010010179, in particular Seq ID No: 1. Other antigens of interest in the field of tuberculosis include Rv3616 and variants thereof, such as disclosed in WO2011092253, for example a natural Rv3616 sequence selected from Seq ID Nos: 1 and 2-7 of WO2011092253 or a modified Rv3616 sequence such as those selected from Seq ID Nos: 161 to 169, 179 and 180 of WO2011092253, in particular Seq ID No: 167. An additional antigen of interest is HBHA, such as disclosed in WO97044463, WO03044048 and WO2010149657.

Tuberculosis antigens are suitably utilised in the form of a polypeptide, but may alternatively be provided in the form of a polynucleotide encoding said polypeptide.

A further antigen that may be employed in accordance with the present invention is derived from Varicella zoster virus (VN). The VZV antigen for use in the invention may be any suitable VZV antigen or immunogenic derivative thereof, suitably being a purified VZV antigen.

The term 'immunogenic derivative' encompasses any molecule which retains the ability to induce an immune response to VZV following administration to man.

Suitable methods for the generation of derivatives are well known in the art and include standard molecular biology techniques as disclosed, for example, in Sambrook et al [Molecular Cloning: A Laboratory Manual, third edition, 2000, Cold Spring Harbor Laboratory Press], such as techniques for the addition, deletion, substitution or rearrangement of amino acids or chemical modifications thereof Derivatives may include, for example, truncations or other fragments.

Derivatives in the context of this invention may be amino acid sequences comprising epitopes, i.e., antigenic determinants substantially responsible for the immunogenic properties of a polypeptide and being capable of eliciting an immune response, in one aspect being T cell epitopes.

The level of immunogenic activity of the immunogenic derivative may be at least about 50%, at least about 70% and at least or greater than about 90% of the immunogenicity for the polypeptide from which it is derived, suitably as assessed by immunoassay techniques described above. In some aspects of the invention immunogenic portions may be identified that have a level of immunogenic activity greater than that of the corresponding full-length polypeptide, e.g., having greater than about 100% or 150% or more immunogenic activity. The VZV antigen may be a glycoprotein, for example the gE antigen (also known as gpl), or immunogenic derivative thereof.

The gE antigen, anchorless derivatives thereof (which are also immunogenic derivatives) and production thereof is described in EP0405867 and references therein [see also Vafai A. Antibody binding sites on truncated forms of varicalla-zoster virus gpl(gE) glycoprotein Vaccine 1994 12:1265-9]. EP192902 also discloses gE and production thereof.

gE may be a truncated gE having the sequence of figure 1 herein, and as disclosed in Virus research, vol 40, 1996 p199 ff. Reference to gE hereinafter includes reference to truncated gE, unless otherwise apparent from the context.

Alternatively, the antigen or antigenic composition may be a derivative of any of the antigens described herein. As used herein the term "derivative" refers to an antigen that is modified relative to its naturally occurring form. Derivatives described in the present disclosure are sufficiently similar to native antigens to retain antigenic properties and remain capable of allowing an immune response to be raised against the native antigen. Whether or not a given derivative raises such an immune response may be measured by a suitable immunological assay such as an ELISA or flowcytometry.

The present disclosure further describes an immunogenic composition as described herein for use in the treatment of disease. For example, the disclosure describes an immunogenic composition for use in the treatment of a disease associated with the antigens described above, such as for use in the treatment of cancer, malaria, tuberculosis or herpes. The cancer may be selected from the group consisting of prostate, breast, colorectal, lung, pancreatic, renal, ovarian or melanoma cancers.

The present disclosure further describes a method of therapy or prophylaxis of cancer, malaria, tuberculosis or herpes in an individual in need thereof comprising the step of providing said individual with an effective amount of an immunogenic composition as described herein.

### Examples

### Example 1 - Initial experiments

### Experiment 1

### Material and methods:

800g of DOPC + 200g of cholesterol dissolved in 4L ethanol in 20L glass flask Dissolution step in water bath at 55°C and agitation with a Rotary evaporator

Evaporation of the Ethanol with water bath temperature of 55°C and following vacuum pressure program:
- 5 min at 600 mbar
- 600 to 210 mbar in 15 min
- 210 mbar during 30 min
- 210 to 135 mbar

### Results:

The dissolution of the lipid mix was successful. The distillation program was interrupted at the last step due to crystallisation of cholesterol

### Discussion:

Pressures used did not allow to avoid cholesterol crystallisation. Experiment 2 performed with new pressure program

### Experiment 2:

### Material and methods:

800g of DOPC + 200g of cholesterol dissolved in 4L ethanol in 20L glass flask Dissolution step in water bath at 55°C and agitation with a Rotary evaporator

Evaporation of the Ethanol with water bath temperature of 55°C and following vacuum pressure program:
- 5 min at 600 mbar
- 600 to 400 mbar in 2 min
- 400 to 230 mbar in 15 min
- 230 mbar during 165 min
- 230 to 20 mbar in 60 min

### Results:

The dissolution of the lipid mix was successful. The distillation program was interrupted after 132 minutes due to cholesterol crystallisation

### Discussion:

Higher Pressures and longer pressure plateaus did not allow to avoid cholesterol crystallisation. Experiment 3 performed with manual setting of the pressure

### Experiment 3:

### Material and methods:

800g of DOPC + 200g of cholesterol + 40g of MPL dissolved in 2L ethanol in 20L glass flask Dissolution step in water bath at 55°C and agitation with a Rotary evaporator

Evaporation of the Ethanol with water bath temperature at 55°C and vacuum pressure set manually in order to maintain the lipid mix temperature at 50°C, controlled by an additional temperature probe placed in the lipid mix:

### Results:

The dissolution of the lipid mix was successful.

Ethanol was successfully evaporated in 200 minutes

### Discussion:

Cholesterol crystallisation was avoided in this experiment by setting the vacuum pressure manually over the time of ethanol distillation. This way of working is not acceptable for an industrial process as this would not lead to a robust process as required for the industry. Ethanol solvent is not appropriate to run the lipidic film process at the scale of 1040g of lipid

### Example 2 - selection of isopropanol

### Material and methods:

3 solvent compared: Ethanol, Isopropanol, Heptane

Cholesterol was progressively added to each solvent at 50°C up to saturation of the solution

Once saturation is achieved, a sample of the solution is taken and the solvent is evaporated with a rotary evaporator and/or in an oven. Dry residue of cholesterol is weighed to determine the solubility value

### Results:

The cholesterol solubility measured at 50°C is respectively 6.7% in ethanol, 4.3% in heptane and 12.1% in isopropanol

### Discussion:

Based on these results, it appeared that Isopropanol could be a good solvent alternative to Ethanol. Lipidic film experiments with isopropanol were consequently initiated.

### Example 3 - Concentrated Liposomes bulk preparation

The concentrated liposome bulk is prepared in 2 steps: the first step is the lipidic film preparation and the second step is the preparation of the concentrated liposomes bulk

### Lipidic film preparation

As a first step to the lipidic film production, DOPC (Dioleoyl phosphatidylcholine), MPL and cholesterol are dissolved sequentially in isopropanol (IPA). First 780 to 820g DOPC and 39 to 41g MPL are suspended in 4L of IPA in a 20-L round-bottom flask placed in a warming bath at 55°C (50-60°C range) and rotated at 75 rpm (70-80 rpm range) at atmospheric pressure for 30 to 45 minutes. Then 195-2059 cholesterol is added to the DOPC/ MPL solution kept in the warming bath at 55°C (50-60°C range) and rotated at 75 rpm (70-80 rpm range) at atmospheric pressure for 60 to 90 minutes to achieve complete dissolution of the cholesterol.

After raw materials dissolution completion, IPA is stripped off under stirring (70-80 rpm range) and reduced pressure gradient in a warming bath at 55°C (50-60°C range) to obtain a film residue. d. The pressure is first dropped from 1000 to 600 in 2 minutes, 600 to 200 mbar in 5 minutes, then from 200 to 50mbar in 30 min. A plateau at 50 mbar is then applied for 60 min. The pressure is subsequently dropped to the final target of O mbar in 10 min. A final drying at O mbar) is performed for 90 min to obtain a lipidic film weight of 1014 to 1080g

The resulting lipidic film is stored for up to 31days at 2 to 8°C if not processed immediately.

The first step to the concentrated liposomes bulk preparation is the lipidic film hydration in a bufferto form a coarse suspension of liposomes. The lipid film is hydrated by adding the first half (9.2 to 9.7 kg) of PBS (9 mM Na₂HPO_{4,} 41 mM KH₂PO_{4,} 100 mM NaCl pH 6.1) in the 20-L round-bottom flask. The flask is rotated at 79-81 rpm in a water bath at room temperature (15-25°C range) for 60 to 120 minutes. The resulting lisposome suspension is then transferred to a stainless steel tank and diluted with the second half (9.2 to 9.7 kg) of the same modified PBS to achieve the target concentration of 52g /L of lipidic film. The lipidic film suspension is post-stirred for 15 minutes at 350 rpm at room temperature (15-25°C range) to get large liposomes.

Following this hydration step, the liposome suspension is homogenized with a high-shear mixer in-line with a high-pressure homogenizer to produce the desired nano-sized liposomes. The liposome suspension (19.5 to 20.5 kg) is recirculated under 2 bar of air feeding pressure, 140 to 160 rpm agitation and a flow rate of 130 to 140 kg/h. A homogenization pressure of 11500 to 13500 psi is applied and the suspension temperature of the product is maintained at 20-25°C in the surging vessel. The homogenization process is stopped after the equivalent of 10 passes through the homogenization system.

The resulting concentrated bulk liposomes is filtered through a 0.22 µm PES membrane in an aseptic (Grade A/class 100) area or isolator. The product is stored in sterile glass containers or HDPE bags at +2 to +S° C for up to 3 years.

### Example 4 - Formulation into adjuvant system AS01

A stainless steel tank vessel is filled with the required amount of water.

Concentrated phosphate buffer at 100mM PO4 and NaCl solution at 1500mM are diluted in the water for injection in order to reach the final concentration of 10mM PO4 and 150mM NaCl in the AS01 formulation. The buffer solution is stirred for 15 to 45 minutes at room temperature.

The concentrated liposomes bulk is added to the buffer solution at the final concentration of 25 to 50µg per dose of AS01. The diluted liposomes bulk is stirred for 15 to 45 min The QS21 liquid bulk is added to the liposomes solution at the final concentration of 25 to 50µg per dose of AS01 to form the AS01 final bulk. The AS01 solution is stirred for 15 to 45 min at room temperature.

The AS01 bulk is filtered through a 0.22µm PES membrane in an aseptic (GradeA/class100) or isolator area. The product is stored in the tank at +2 to +8°C for up to 30 days before filling into sterile glass vials.

## Claims

1. A method for producing a lipidic film comprising the steps of:
(i) dissolving a lipid mix in isopropanol to form a homogeneous mix; and
(ii) removing the isopropanol from the homogeneous mix to form a lipidic film,
wherein the lipid mix comprises a lipid, cholesterol and a lipopolysaccharide, wherein the total amount of lipid mix dissolved in isopropanol is 200g or more

2. The method for producing a lipidic film according to claim 1, wherein the lipopolysaccharide is monophosphoryl lipid A(MPL).

3. The method for producing a lipidic film according to claim 1, wherein the lipopolysaccharide is 3-deacylated monophoshoryl lipid A(3D-MPL).

4. The method for producing a lipidic film according to any one of claims 1 to 3, wherein the solvent is removed from the homogeneous mix by evaporation.

5. The method for producing a lipidic film according to claim 4, wherein the solvent is removed from the homogeneous mix by vacuum distillation.

6. The method for producing a lipidic film according any one of claims 1 to 5, wherein the lipid comprises dioleoylphosphatidylcholine (DOPC).

7. A method for producing liposomes comprising:
a) producing a lipidic film according to the method of any one of claims 1 to 6;
b) hydrating the lipidic film with a hydrating solution to form a coarse liposome suspension;
c) reducing size of the coarse liposome suspension produced in step b) with high shear and high pressure homogenizer to form liposomes

8. The method according to claim 7, wherein the hydrating solution comprises buffer

9. The method according to claim 8, wherein the buffer is a phosphate buffer.

10. The method according to anyone of claims 7 to 9, wherein the method comprises an additional step d) sterilising the liposomes.

## Patentansprüche

1. Verfahren zur Erzeugung eines Lipidfilmes, enthaltend die Schritte:
i) Auflösen einer Lipidmischung in Isopropanol, zur Bildung einer homogenen Mischung, und
ii) Entfernung des Isopropanols von der homogenen Mischung, zur Bildung eines Lipidfilmes,
worin die Lipidmischung ein Lipid, Cholesterin und ein Lipopolysaccharin enthält, worin die Gesamtmenge der Lipidmischung, die in Isopropanol aufgelöst ist, 200 g oder mehr ist.

2. Verfahren zur Erzeugung eines Lipidfilmes nach Anspruch 1, worin das Lipopolysaccharid Monophosphoryllipid A (MPL) ist.

3. Verfahren zur Erzeugung eines Lipidfilmes nach Anspruch 1, worin das Lipopolysaccharid 3-deacyliertes Monophosphoryllipid A (3D-MPL) ist.

4. Verfahren zur Erzeugung eines Lipidfilmes nach einem der Ansprüche 1 bis 3, worin das Lösungsmittel von der homogenen Mischung durch Verdampfung entfernt wird.

5. Verfahren zur Erzeugung eines Lipidfilmes nach Anspruch 4, worin das Lösungsmittel von der homogenen Mischung durch Vakuumdestillation entfernt wird.

6. Verfahren zur Erzeugung eines Lipidfilmes nach einem der Ansprüche 1 bis 5, worin das Lipid Dioleoylphosphatidylcholin (DOPC) enthält.

7. Verfahren zur Erzeugung von Liposomen, enthaltend:
a) Produktion eines Lipidfilmes nach dem Verfahren nach einem der Ansprüche 1 bis 6,
b) Hydratisieren des Lipidfilmes mit einer Hydratisierlösung, zur Bildung einer groben Liposomsuspension,
c) Reduktion der Größe der groben Liposomsuspension, erzeugt im Schritt b), mit einem Homogenisator mit hoher Scherkraft und hohem Druck, zur Bildung von Liposomen.

8. Verfahren nach Anspruch 7, worin die Hydratisierlösung einen Puffer enthält.

9. Verfahren nach Anspruch 8, worin der Puffer ein Phosphatpuffer ist.

10. Verfahren nach einem der Ansprüche 7 bis 9, worin das Verfahren einen zusätzlichen Schritt d) zum Sterilisieren der Liposome enthält.

## Revendications

1. Procédé pour produire un film lipidique comprenant les étapes de:
(1) dissolution d'un mélange de lipides dans l'isopropanol pour former un mélange homogène; et
(2) retrait de l'isopropanol du mélange homogène pour former un film lipidique,
dans lequel le mélange de lipides comprend un lipide, du cholestérol et un lipopolysaccharide, dans lequel la quantité totale de mélange de lipides dissous dans l'isopropanol est 200 g ou plus.

2. Procédé pour produire un film lipidique selon la revendication 1, dans lequel le lipopolysaccharide est le monophosphoryl lipide A (MPL).

3. Procédé pour produire un film lipidique selon la revendication 1, dans lequel le lipopolysaccharide est le monophosphoryl lipide A 3-désacylé (3D-MPL).

4. Procédé pour produire un film lipidique selon l'une quelconque des revendications 1 à 3, dans lequel le solvant est retiré du mélange homogène par évaporation.

5. Procédé pour produire un film lipidique selon la revendication 4, dans lequel le solvant est retiré du mélange homogène par distillation sous vide.

6. Procédé pour produire un film lipidique selon l'une quelconque des revendications 1 à 5, dans lequel le lipide comprend de la dioléoylphosphatidyl-choline (DOPC).

7. Procédé pour produire des liposomes comprenant:
(a) la production d'un film lipidique selon le procédé de l'une quelconque des revendications 1 à 6;
(b) l'hydratation du film lipidique avec une solution hydratante pour former une suspension de liposomes grossière;
(c) la réduction de la taille de la suspension de liposomes grossière produite dans l'étape b) avec un homogénéisateur à haut cisaillement et haute pression pour former des liposomes.

8. Procédé selon la revendication 7, dans lequel la solution hydratante comprend un tampon.

9. Procédé selon la revendication 8, dans lequel le tampon est un tampon phosphate.

10. Procédé selon l'une quelconque des revendications 7 à 9, dans lequel le procédé comprend une étape supplémentaire d) de stérilisation des liposomes.
